# EUROPÄISCHE PATENTSCHRIFT

(11) **EP 0 476 552 B1**
(45) Veröffentlichungstag und Bekanntmachung des Hinweises auf die Patenterteilung: **28.12.1994**
(21) Anmeldenummer: 91115621.4
(22) Anmeldetag: 14.09.1991
(51) Int. Cl.: G02B 21/24, F16M 11/12

(54) **Schwenkeinrichtung für Tragvorrichtungen für optische Beobachtungsgeräte**
Slewing mechanism for supporting devices for optical observation instruments
Mécanisme vireur pour dispositifs de support pour instruments d'observation optiques

(30) Priorität: 19.09.1990 DE 4029638
(43) Veröffentlichungstag der Anmeldung: 25.03.1992
(73) Patentinhaber: Firma Carl Zeiss, D-73446 Oberkochen (DE)
(72) Erfinder: Heller, Rudolf, CH-8052 Zürich (CH)

(56) Entgegenhaltungen:
- EP-A- 0 293 228
- WO-A-81/03054
- CH-A- 482 439
- DE-A- 3 147 836

## Beschreibung

Die Erfindung betrifft eine Schwenkeinrichtung für Tragvorrichtungen für optische Beobachtungsgeräte, beispielsweise Operationsmikroskope, mit einer von einer Befestigungsstelle sich zu einem Objektpunkt erstreckenden, mittels einer Höhenverstellung und mindestens eines Gelenkparallelogrammes in drei Koordinatenachsen verstellbaren Verstelleinrichtung und mit einer mit der Verstelleinrichtung verbundenen Schwenkeinrichtung.

Aus der CH-A-482 439 ist eine Tragvorrichtung für ein Operationsmikroskop bekannt, die aus der unlösbaren Verbindung einer in drei Koordinatenachsen verstellbaren Verstelleinrichtung mit einer um zwei Drehachsen schwenkbaren Schwenkeinrichtung besteht.

Diese bekannte Tragvorrichtung ist jedoch sperrig in ihren Ausmaßen und für Anwendungsgebiete, bei denen ein optisches Beobachtungsgerät nur in der Höhe und in zwei Koordinatenachsen translatorisch verstellt werden soll, zu aufwendig. Außerdem befriedigt eine Schwenkung des Operationsmikroskopes um nur zwei Drehachsen nicht alle Forderungen des Chirurgen, z.B. bei schwierigen Gehirnoperationen.

Der Erfindung liegt deshalb die Aufgabe zugrunde, eine Schwenkeinrichtung anzugeben, die um drei Drehachsen schwenkbar und mit in drei Koordinatenachsen verstellbaren Verstelleinrichtungen zu einer vielseitig anwendbaren Tragvorrichtung für optische Beobachtungsgeräte kombinierbar ist.

Die Lösung dieser Aufgabe erfolgt erfindungsgemäß durch die Merkmale des Anspruchs 1.

In einer vorteilhaften Ausgestaltung der Erfindung beträgt der von der zweiten und dritten Drehachse eingeschlossene Winkel mindestens 30° und die Drehachse des Balancierarms verläuft parallel zur Drehachse des Schwenkarms.

Durch eine Antriebsverbindung des Schwenkarms mit dem Balancierarm wird die mit der Schwenkeinrichtung ausgeführte Bewegung erleichtert.

Die Antriebsverbindung zwischen dem Schwenkarm und dem Balancierarm kann beispielsweise aus je einem an den Drehlagern befestigten und durch einen Riemen oder eine Kette verbundenen Kettenrad oder aus einem an sich bekannten Kegelradtrieb bestehen.

Ein Ausführungsbeispiel der Erfindung ist in der Zeichnung dargestellt und wird im folgenden näher beschrieben.

Es zeigen
- Figur 1: eine Tragvorrichtung, bestehend aus der Kombination einer Schwenkeinrichtung mit einer in drei Raumkoordinaten verstellbaren Verstelleinrichtung;
- Figur 2: die Schwenkeinrichtung der in Figur 1 gezeigten Tragvorrichtung mit durch Strichlinien angedeutetem Beobachtungsgerät;
- Figur 3: eine unvollständige Schnittdarstellung der Schwenkeinrichtung mit den Drehlagern ihrer Schwenkarme.

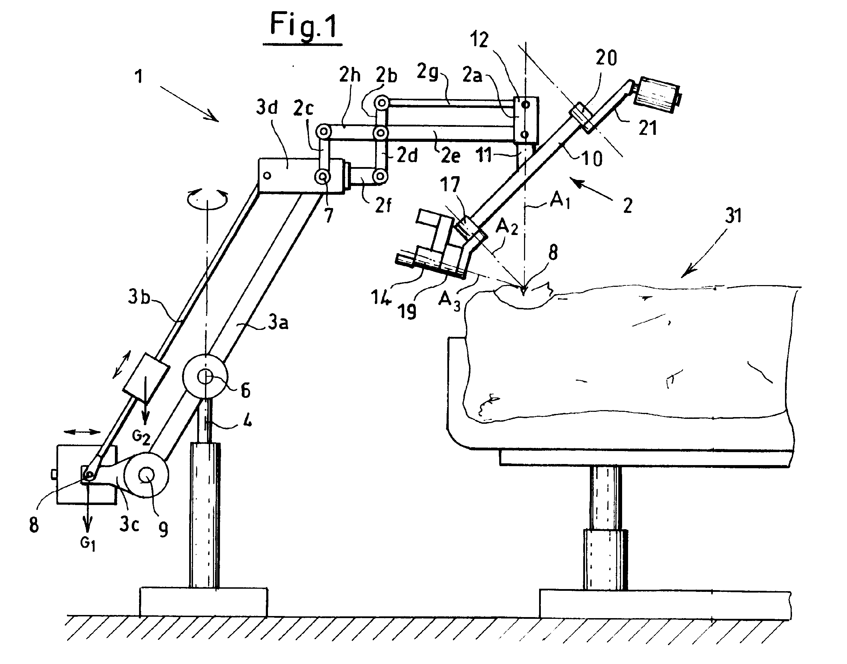

Die Figur 1 zeigt eine in drei Koordinatenrichtungen verstellbare Verstelleinrichtung (1) in Verbindung mit der erfindungsgemäßen Schwenkeinrichtung (2). Die Verstelleinrichtung (1) besteht aus einer vertikalen, in der Höhe verstellbaren Trägersäule (4), mit der über ein Drehlager (6) ein Gelenkparallelogramm (3a-3d) verbunden ist. Eine Seite des Gelenkparallelogramms (3a-3d) ist als zweiarmiger Hebel (3a) ausgeführt, der in einem Drehlager (6) drehbar ist. Die Gegenseite zum Hebel (3a) ist als Stange (3b) ausgeführt, auf der ein Gewicht (G2) axial verschiebbar angeordnet ist. Im Verbindungsgelenk (9) des Hebels (3a) ist parallel zur Parallelogrammseite (3c) ein weiteres verschiebbares Ausgleichsgewicht (G1) angeordnet. Das Verbindungsgelenk (7) des Hebels (3a) mit seiner benachbarten Parallelogrammseite (3d) ist mit einem weiteren Parallelogramm (2a-2h) verbunden, das über ein Drehlager (12) die Schwenkvorrichtung (2) trägt.

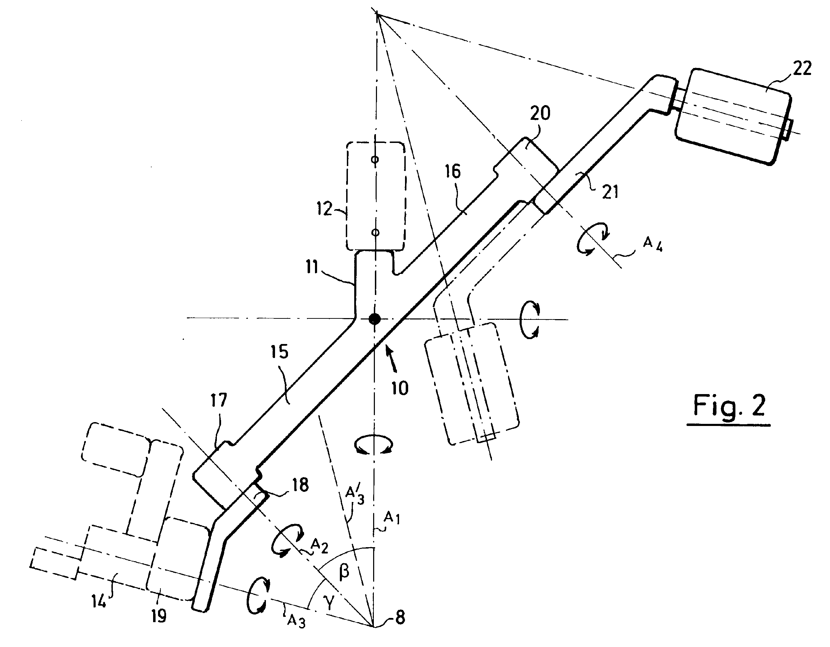

Die Funktionsweise der Schwenkvorrichtung (2) ist aus der Figur 2 ersichtlich.

Sie hat einen Tragbalken (10), der um seine erste geometrische Achse (A₁) drehbar ist, indem er einen abstehenden Lagerschaft (11) aufweist, der in einem an der ersten Trageinrichtung (1) befestigten Drehlager (12) gehalten ist. Dadurch, daß dieser Tragbalken (10) mit seinem zu dem Beobachtungsgerät (14) hingerichteten Arm (15) von dem Bereich, in dem er mit der ersten Trageinrichtung (1) verbunden ist, nach unten geneigt verläuft, kann das Beobachtungsgerät (14) unterhalb der Trageinrichtung (1) angebracht sein, und es ergibt sich ein großer Verstellbereich, in dem eine Behinderung des Operateurs durch Gestängeteile der Tragvorrichtung weitmöglichst vermieden wird.

Eine Verstellbarkeit des Neigungswinkels des Beobachtungsgerätes (14) gegenüber einer Horizontalebene ohne Veränderung der Fokussierung und bei allseitiger Beobachtungsmöglichkeit um die Achse (A₁) ergibt sich durch einen am Ende des Armes (15) in einem Drehlager (17) gelagerten Schwenkarm (18), an dessen Ende das Beobachtungsgerät (14) in einem Drehlager (19) gehalten ist und dadurch, daß die Drehachsen (A₁, A₂, A₃) des Tragbalkens (10), des Schwenkarmes (18) und des Beobachtungsgerätes (14) sich im Beobachtungspunkt (8) schneiden. Dabei schließen die Drehachsen (A₂, A₃) einen Winkel γ ein, der vorzugsweise mindestens 30° beträgt und nicht größer ist als der Winkel β zwischen den Drehachsen (A₁, A₂), so daß die Achse (A₃) des Beobachtungsgerätes bis in Koinzidenz mit der Drehachse (A₁) des Tragbalkens (10) aufwärts schwenkbar ist. Eine Schwenkstellung ist durch die gestrichelt eingezeichnete Achsstellung (A'₃) angedeutet.

Eine leichte Beweglichkeit des Beobachtungsgerätes (14) um die Drehachsen (A₁, A₂, A₃) ergibt sich durch eine zu seiner Bewegung synchrone Ausbalancierung, indem der das Beobachtungsgerät (14) tragende Schwenkarm (18) in Antriebsverbindung steht mit einem am anderen Arm (16) des zweiarmig ausgeführten Tragbalkens (10) in einem Drehlager (20) gehaltenen Balancierarm (21). An seinem äußeren Ende trägt der Balancierarm (21) zu dem Beobachtungsgerät (14) ein Gegengewicht (22), dessen Masse von den Längenverhältnissen von Balancierarm (21), Schwenkarm (18) und den Armen (15, 16) des Tragbalkens (10) bestimmt wird.

Die Antriebsverbindung zwischen dem Schwenkarm (18) und dem Balancierarm (21) besteht beispielsweise aus einer endlosen Kette (23), die in je ein Kettenrad (24, 25) eingreift, das auf dem jeweiligen Wellenzapfen (26, 27) des jeweiligen Drehlagers (17, 20) befestigt ist.

Es ist auch denkbar, andere bekannte Antriebsverbindungen zu verwenden, beispielsweise einen Kegelradantrieb.

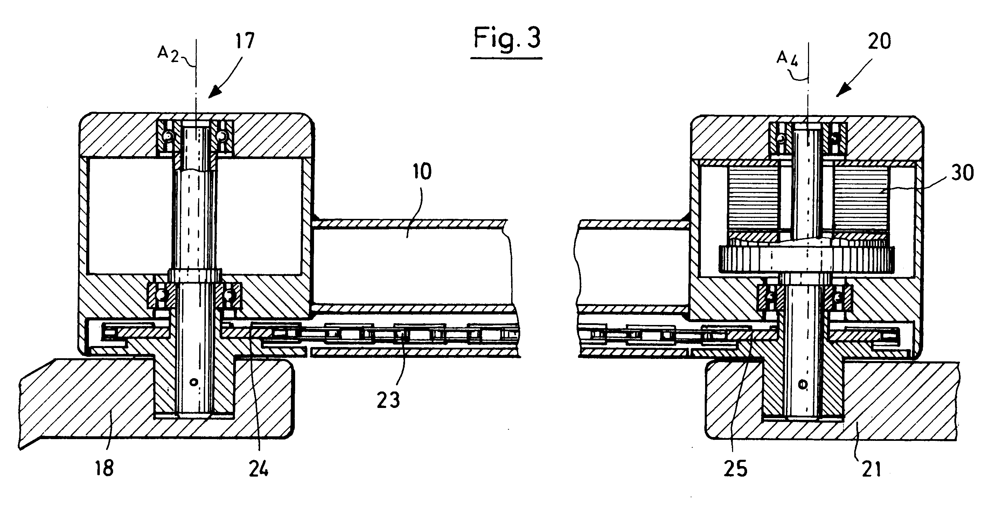

Für die Fixierung der mittels der zweiten Trageinrichtung (2) eingestellten Beobachtungsposition, z.B. durch Führung mittels eines Mundgriffes während einer mikrochirurgischen Operation, sind die Drehlager (12, 17, 19, 20) oder einige dieser Drehlager (im Ausführungsbeispiel der Figur 3 das Drehlager (20)) mittels einer elektromagnetischen Bremse (30) feststellbar, die z.B. durch Fußbetätigung aktiviert wird.

## Patentansprüche

1. Schwenkeinrichtung (2) zur Halterung eines optischen Beobachtungsgeräts (14), beispielsweise eines Operationsmikroskops, an der Verstelleinrichtung (1) einer Tragevorrichtung, wobei die Ausrichtung des Beobachtungsgeräts auf einen bestimmten Beobachtungspunkt (8) vorgesehen ist, und das Beobachtungsgerät um eine durch diesen Beobachtungspunkt verlaufende Achse (A₃) drehbar ist, mit einem Tragbalken (10) mit einem ersten und einem zweiten Ende, der über ein erstes Drehlager (12) mit der Verstelleinrichtung (1) verbunden und um eine erste durch dieses Drehlager verlaufende Drehachse (A₁) drehbar ist, welche die Längsachse des Tragbalkens nicht rechtwinklig und im Bereich zwischen dem ersten und dem zweiten Ende des Tragbalkens schneidet, mit einem Schwenkarm (18), der einerseits am ersten Ende des Tragbalkens (10) über ein zweites Drehlager (17) mit diesem verbunden und um eine zweite durch das zweite Drehlager (17) verlaufende Drehachse (A₂) drehbar ist, und an dem andererseits die Montage des Beobachtungsgeräts (14) vorgesehen ist, wobei das erste und zweite Drehlager (12, 17) so angeordnet sind, und der Schwenkarm so ausgestaltet ist, daß die erste und die zweite Drehachse (A₁, A₂) sowie die Drehachse des am Schwenkarm montierten Beobachtungsgeräts (A₃) sich im Beobachtungspunkt (8) schneiden, und mit einem Balancierarm (21) mit einem Gegengewicht (22), der mit dem zweiten Ende des Tragbalkens (10) über ein weiteres Drehlager (20) verbunden und um eine zur Längsachse des Tragbalkens senkrecht stehende und durch dieses Drehlager (20) verlaufende Drehachse (A₄) drehbar ist.

2. Schwenkeinrichtung für Tragvorrichtungen nach Anspruch 1, dadurch gekennzeichnet, daß der von der zweiten und dritten Drehachse eingeschlossene Winkel γ mindestens 30° beträgt.

3. Schwenkeinrichtung für Tragvorrichtungen nach Anspruch 2, dadurch gekennzeichnet, daß die Drehachse (A₄) des Balancierarms parallel sur zweiten Drehachse (A₂) verläuft.

4. Schwenkeinrichtung für Tragvorrichtungen nach Anspruch 3, dadurch gekennzeichnet, daß der Schwenkarm (18) in Antriebsverbindung mit dem Balancierarm (21) steht.

5. Schwenkeinrichtung für Tragvorrichtungen nach Anspruch 4, dadurch gekennzeichnet, daß die Antriebsverbindung zwischen dem Schwenkarm (18) und dem Balancierarm (21) aus je einem an den Drehlagern (17, 20) befestigten und durch einen Riemen oder eine Kette (29) verbundenem Kettenrad (24, 25) besteht.

6. Schwenkeinrichtung für Tragvorrichtungen nach Anspruch 4, dadurch gekennzeichnet, daß die Antriebsverbindung zwischen dem Schwenkarm (18) und dem Balancierarm (21) aus einem Kegelradantrieb besteht.

7. Schwenkeinrichtung für Tragvorrichtungen nach einem der Ansprüche 4 oder 5, dadurch gekennzeichnet, daß mindestens eines der Drehlager (12, 17, 19, 20) mittels einer elektromagnetischen Bremse (30) feststellbar ist.

## Claims

1. Pivoting device (2) for supporting an optical observation unit (14), e.g. surgical microscopes, said pivoting device (2) being connected with an adjustment mechanism (1) of a supporting device,
said optical observation unit (14) being adjustable to a certain observation point (8),
said optical observation unit (14) being rotatable about an axis (A₃) which extends from the observation point (8), said pivoting device (2) comprising a supporting bar (10) with a first end and a second end, said supporting bar (10) being connected via a first pivot bearing (12) with said adjustment mechanism (1) and being rotable around an axis (A₁), said axis (A₁) extends from said first pivot bearing (12) and intersects the longitudinal axis of said supporting bar (10) in an angle which is different from 90° and said axis (A₁) intersects said supporting bar (10) between said first and second end,
a swivel arm (18) being connected with said first end of said supporting bar (10) via a second pivot bearing (17) and being rotatable around a second axis (A₂) which intersects said second pivot bearing (17) and said optical observation unit (14) being mounted on that swivel arm (18),
said first and said second swivel bearing (12, 17) being mounted and said swivel arm (18) being constructed so that said first and second axis (A₁, A₂) and said axis (A₃) of the optical observation unit (14) which is mounted at the swivel arm (18) intersect in the observation point (8), a balance arm (21) which supports a counterweight (22), said balance arm (21) being connected with the second end of said supporting bar (10) via a further pivot bearing (20), said balance arm (21) being rotatable around a further axis (A₄) which extends perpendicular from the longitudinal axis of said supporting bar (10) and which intersects said pivot bearing (20).

2. Pivoting device for supporting devices according to claim 1, characterized in that the angle (γ) subtended by said second axis and said third axis of rotation is at least 30°.

3. Pivoting device for supporting devices in accordance with claim 2, characterized in that the axis of rotation (A₄) of the balance arm extends parallel to the second axis of rotation (A₂).

4. Pivoting device for supporting devices in accordance with claim 3, characterized in that a drive connection exists between the swivel arm (18) and the balance arm (21).

5. Pivoting device for supporting devices in accordance with claim 4, characterized in that the drive connection between the swivel arm (18) and the balance arm (21) comprises sprocket wheels (24, 25) which are mounted to the pivot bearings (17, 20) respectively and connected by a belt or a chain (29).

6. Pivoting device for supporting devices in accordance with claim 4, characterized in that the drive connection between the swivel arm (18) and the balance arm (21) is a bevel-gear drive.

7. Pivoting device for supporting devices in accordance with one of the claims 4 or 5, characterized in that at least one of the pivot bearings (12, 17, 19, 20) can be locked in position by means of an electromagnetic brake (30)

## Revendications

1. Dispositif de pivotement (2) destiné à monter un appareil optique d'observation (14), un microscope d'opération p.ex., sur le dispositif de réglage (1) d'un ensemble porteur, l'appareil d'observation étant aligné sur un point observé (8) et orientable autour d'un axe (A₃) passant par ledit point observé, comportant un bras support à deux extrémités qui est solidaire du dispositif de positionnement (1) par l'intermédiaire d'un premier coussinet de pivotement (12) et qui est orientable autour d'un premier axe de rotation (A₁) passant par ledit coussinet de pivotement et coupant l'axe longitudinal du bras support entre ses deux extrémités selon un tracé non orthogonal, comportant en outre un bras pivotant (18) qui est d'une part solidaire du bras support (10) par l'intermédiaire d'un deuxième coussinet de pivotement (17) disposé sur la première extrémité de celui-ci et orientable autour d'un deuxième axe de rotation (A₂) passant par ledit coussinet de pivotement (17), et qui est d'autre part prévu pour le montage de l'appareil d'observation (14), le premier et le deuxième coussinet de pivotement (12, 17) et le bras pivotant étant respectivement disposés et réalisé de manière à ce que lesdits axes de rotation (A₁, A₂) et l'axe de rotation (A₃) de l'appareil d'observation monté sur le bras pivotant se recoupent dans le point observé (8), comportant enfin un bras d'équilibrage (21) équipé d'un contrepoids (22), qui est solidaire du bras support (10) par l'intermédiaire d'un autre coussinet de pivotement (20) disposé sur la seconde extrémité de celui-ci et orientable autour d'un axe de rotation (A₄) qui est perpendiculaire à l'axe longitudinal du bras support et qui passe par ledit coussinet de pivotement (20).

2. Dispositif de pivotement pour ensembles porteurs selon la revendication 1, caractérisé en ce que l'angle γ formé par les axes de rotation A₂ et A₃ est égal ou supérieur à 30°.

3. Dispositif de pivotement pour ensembles porteurs selon la revendication 2, caractérisé en ce que l'axe de rotation (A₄) du bras d'équilibrage est parallèle au deuxième axe de rotation (A₂).

4. Dispositif de pivotement pour ensembles porteurs selon la revendication 3, caractérisé en ce que le bras d'équilibrage (21) est asservi au système d'entraînement du bras pivotant (18).

5. Dispositif de pivotement pour ensembles porteurs selon la revendication 4, caractérisé en ce que la transmission entre le bras pivotant (18) et le bras d'équilibrage (21) est assurée par des roues dentées (24, 25) montées sur les coussinets de pivotement (17, 20) et reliées entre elles par une courroie ou par une chaîne (29).

6. Dispositif de pivotement pour ensembles porteurs selon la revendication 4, caractérisé en ce que la transmission entre le bras pivotant (18) et le bras d'équilibrage (21) est assurée par une commande à roues coniques.

7. Dispositif de pivotement pour ensembles porteurs selon l'une quelconque des revendications 4 à 6, caractérisé en ce que l'un au moins des coussinets de rotation (12, 17, 19, 20) soit immobilisable à l'aide d'un frein électromagnétique (30).
